# EUROPEAN PATENT SPECIFICATION

(11) **EP 0 468 187 B1**
(45) Date of publication and mention of the grant of the patent: **18.03.1998**
(21) Application number: 91109708.7
(22) Date of filing: 13.06.1991
(51) Int. Cl.: C07D 265/18, C07D 265/26, C07D 401/04, C07D 401/06, C07D 403/12, C07D 405/14, C07D 413/06, C07D 413/12, C07D 413/14, C07D 419/12, C07D 471/04, C07D 475/02, C07D 487/04, C07D 495/04, C07D 498/04, A61K 31/165

(54) **Cyclic amide derivatives**
Derivate von cyclischen Amiden
Dérivés d'amides cycliques

(30) Priority: 15.06.1990 JP 157134/90
(43) Date of publication of application: 29.01.1992
(60) Divisional application: 95108191.8
(73) Proprietor: Eisai Co., Ltd., Tokyo (JP)
(72) Inventor: Sugimoto, Hachiro, Ushiku-shi, Ibaralki (JP); Yonaga, Masahiro, Tsukuba-shi, Ibaraki (JP); Karibe, Norio, Moriyamachi, Kitashouma-gun, Ibaraki (JP); Iimura, Youichi, Tsukuba shi, Ibaraki (JP); Nagato, Satoshi, Tsukuba-shi, Ibaraki (JP); Sasaki, Atsushi, Tsukuba-shi, Ibaraki (JP); Yamanishi, Yoshiharu, Ryugasaki-shi, Ibaraki (JP); Ogura, Hiroo, MD20814 (US); Kosasa, Takashi, Tsukuba-shi, Ibaraki (JP); Uchikoshi, Kumi, Tsuchiura-shi, Ibaraki (JP); Yamatsu, Kiyomi, Kamakura-shi, Kanagawa (JP)
(74) Representative: Hansen, Bernd, Dr. Dipl.-Chem.

(56) References cited:
- EP-A- 0 004 793
- EP-A- 0 013 071
- EP-A- 0 015 138
- EP-A- 0 026 749
- EP-A- 0 047 990
- EP-A- 0 049 173
- EP-A- 0 058 055
- EP-A- 0 065 229
- EP-A- 0 091 511
- EP-A- 0 107 930
- EP-A- 0 138 198
- EP-A- 0 170 213
- EP-A- 0 204 349
- EP-A- 0 229 391
- EP-A- 0 259 793
- EP-A- 0 269 968
- EP-A- 0 296 560
- EP-A- 0 304 330
- EP-A- 0 326 106
- EP-A- 0 344 577
- EP-A- 0 351 282
- WO-A-80/00024
- DE-A- 2 639 718
- US-A- 3 494 932
- US-A- 4 066 772
- J.MED CHEM, Vol 32, 1989, pages 1921-1926, R.A.GLENNON et al., N-(Phthalimidoalkyl derivatives of serotonergic agents
- PATENT ABSTRACTS OF JAPAN, vol 15 No 104, (C-814)(4632), 13 March 1991 & JP-A-32155 (TAKEDA CHEM IND LTD) 08.01.1991
- CHEM PHARM BULL, vol 35, No 9, 1987 pages 3558-3567, T.TAKEDA et al.
- CHEM PHARM BULL, vol 34, No 3, 1986, pages 1032-1038, M.NIITSU et al
- HELVETICA CHIMICA ACTA, vol 64, No 40, fasc 2, 1981, pages 399-406, C.HEIDELBERGER et al
- J.HETEROCYCLIC CHEM., vol 20, No 5, September-October 1983, pages 1271-1275, R.GRANADOS et al
- PATENT ABSTRACTS OF JAPAN, Vol 14, No 435 (C760)(4378), 8 September 1990 & JP-A-2169569 (EISAI CO LTD) 29.06.1990

## Description

The present invention relates to a new cyclic amide derivative having excellent medicinal effects.

### [Prior Art]

With a rapid increase in the population of the aged, the establishment of medical treatment for senile dementia such as Alzheimer's disease is eagerly demanded.

Although various treatments of the senile dementia with medicineswere have been attempted, no medicine essentially effective for these diseases has been developed as yet.

Various investigations are in progress for the purpose of developing therapeutic agents for these diseases. Particularly, the development of acetylcholine precursors and acetylcholine esterase inhibitors has been proposed, since Alzheimer's disease is attended with cerebral cholinergic hypofunction, and they are on actual test. Typical examples of the anticholinergic esterase inhibitors include physostigmine and tetrahydroaminoacridine. EP-A-326 106 relates to a cholinesterase inhibiting agent which contains an alkylene diamine of the formula 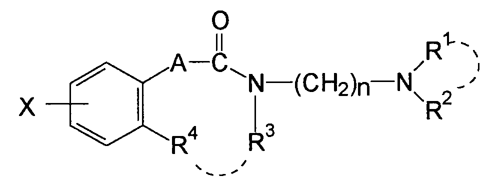 wherein R¹ and R² each independently is a hydrogen atom or a hydrocarbon residue which may optionally be substituted, or R¹ and R² combinedly form, together with the adjacent nitrogen atom, a condensed heterocyclic group, R³ is a hydrogen atom or a hydrocarbon residue or an acyl group, each of which may optionally be substituted and R⁴ is a hydrogen atom, or R³ and R⁴ combinedly form a group of the formula 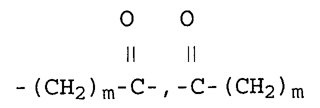 or -(CH₂)ₘ₊₁-(m being 0,1 or 2), A is -CH₂)_{ℓ} (ℓ being 0.1 or 2) or -CH=CH-, X is a substituent or substituents and n is an integer of 4 to 7, or a pharmaceutically acceptable salt thereof and a pharmaceutically acceptable carrier.

EP-A-296 560 relates to piperidine derivatives which have acetyl cholinesterase activity and comprise preferably a monovalent or divalent group, in which the phenyl may have a substituent(s), selected from the group consisting of (1) indanyl, (2) indanonyl, (3) indenyl, (4) indenonyl, (5) indanedionyl, (6) tetralonyl, (7) benzosuberonyl, (8) indanolyl and (9) C₆H₅-CO-CH(CH₃)-.

The examples involve only N-benzyl substituted piperidines.

EP-A-229 391 relates to N-substituted piperidine derivatives, comprising in particular a bicyclic amid bearing two keto functions such as phtalimide, 2,3-pyrazine dicarboxylic acide imide, 2,4-(1H, 3H) quinazolidione. In the concrete examples the piperidine substituent is benzyl or alkyl which may be substituted or pyridyl. The N-substituted piperidine derivatives show Acetylcholin esterase inhibitory activity.

However, they have defects that their effects are yet insufficient and that they have adverse reactions unfavorably. Thus there are no decisive therapeutic agents at present.

### ( Summary of the invention )

Under these circumstances, the inventors have made intensive investigations of various compounds for a long period of time for the purpose of developing a medicine having a high safety and a long-lasting effect.

As the result, the inventors have found that the purpose can be attained by using a derivative of the formula (Ia) given below.

In particular, the compound of the present invention represented by the structural formula (Ia) given below has the features of having a potent, highly selective antiacetylcholine esterase activity to increase the quantity of acetylcholine in the brain, of being effective for the therapy of models of the disturbance of memory, of showing an effect that lasts for a longer time and of having a higher safety than those of physostigmine frequently used in this field. The present invention is thus highly valuable.

The compound of the present invention has been found on the basis of acetylcholine esterase inhibition and, therefore, it is effective for the treatment and prevention of various diseases due to insufficiency of the central choline functions, i.e. lack of acetylcholine as the neurotransmitter in vivo.

Typical examples of such diseases include dementias represented by Alzheimer's presbyophrenia. They include also Huntington's chorea, Pick's disease and tardive dyskinesia.

Therefore, objects of the present invention are to provide a new piperidine derivative usable as a medicine and particularly effective for the treatment and prevention of central nervous diseases, to provide a process for producing this new piperidine derivative and to provide medicines comprising the piperidine derivative as the active ingredient.

The invention provides a cyclic amide derivative having the formula (Ia) or a pharmacologically acceptable salt thereof:

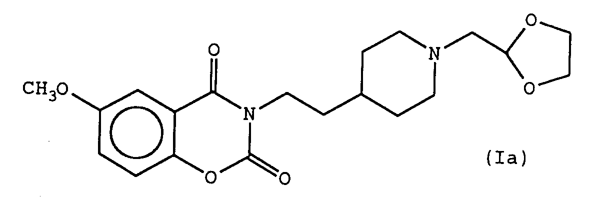

The invention further relates to the pharmacological use of the cyclic amide compound having the formula (Ia) and therefore provides a phamaceutical composition comprising a pharmacologically effective amount of the cyclic amide derivative as defined above and a pharmacologically acceptable carrier and then the use thereof for the preparation of a pharmaceutical useful for preventing and treating diseases due to insufficiency of the central choline functions.

The compound having the formula (Ia) can be produced as shown below.

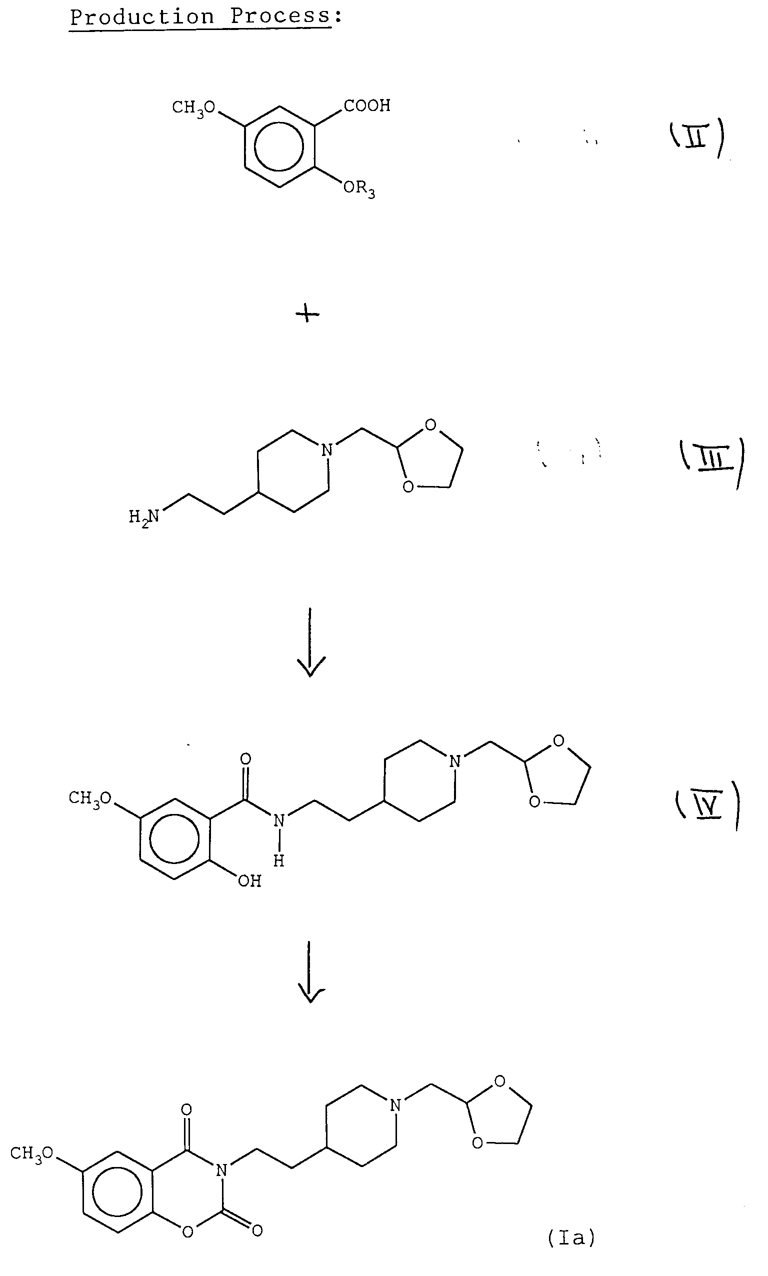

Namely, a salicylic acid derivative of the formula (II) and an amine of the formula (III) are condensed with, for example, 1,1-carbonyldiimidazole in a solvent such as tetrahydrofuran to give a compound (IV) after the protective group R₃ (e.g. benzyl) is removed therefrom by catalytic reduction conducted in the presence of Pd-C or the like in a solvent such as methanol. This compound is reacted with 1,1-carbonyldiimidazole in a solvent such as tetrahydrofuran to give compound (Ia) which is the intended compound.

The compound of the formula (Ia) or acid addition salts thereof produced as described above are effective for the treatment of various senile dementias, particularly Alzheimer's disease.

The following results of pharmacological tests will illustrate the usefulness of the compound of the formula (Ia) and acid addition salts thereof.

### Experimental Example 1

### Acetylcholine esteraze inhibition effect in vitro:

The esterase activity was determined by using a mouse brain homogenate as the acetylcholine esterase source by the method of Ellmann et al¹⁾.
Acetylthiocholine as the substrate, the sample and DTNB were added to the mouse brain homogenate and they were incubated. A yellow product formed by the reaction of the produced thiocholine with DTNB was determined on the basis of a change in absorbance at 412 nm to determine the acetylcholine esterase activity.
1) Ellman, G.L., Courtney, K.D., Andres, V. and Featherstone, R.M., (1961) Piochem. Pharmacol., 7, 88 - 95.

The acetylcholine esterase inhibiting activity of the sample was expressed in terms of 50% inhibiting concentration (IC₅₀).

For example 1 (compound Ia) an IC₅₀-value of 124,5 nM was observed.

The cyclic amide derivatives of the present invention has such features that the structure thereof is quite different from that of ordinary acetylcholine esterase inhibitors, that it has a patent acetylcholine esterase inhibiting effect, that the difference between the intended effect and adverse effect thereof is quite large, that the effect lasts for a long time, that it is a quite stable compound having a high water solubility, which is advantageous from the viewpoint of formulation, that its utilization in vivo is high, that it is substantially free from the first pass effect and that it has a high migration rate into the brain.

Thus the object of the present invention is to provide a new compound effective for the treatment of various dementias and sequelae of cerebral blood vessel disorders, process for producing this compound and a new medicine containing this compound as the active ingredient.

The compound of the present invention is effective for the treatment, prevention, remission, improvement, etc. of senile dementias; particularly cerebral blood vessel disorders due to Alzheimer's presbyophrenia, cerebral stroke (cerebral hemorrhage or cerebral infarction), arteriosclerosis and an external wound of the head; and inattentiveness, disturbance of speech, weakened volition, emotional disorder, inability to fix, hallucination-delusion state and behavior changes owing to sequelae of cerebritis and cerebral palsy.

The compound of the present invention having a potent, highly selective anticholine esterase inhibiting effect is useful as a medicine having such an effect.

The compound of the present invention is effective for the treatment of Alzheimer's presbyophrenia as well as Huntington's chorea, Pick's disease and tardive dyskinesia.

When the compound of the present invention is used as a medicine for such a disease, it is given orally or parenterally. Usually, it is given by parenteral administration such as intravenous, subcutaneous or intramuscular injection or in the form of suppositories or sublingual tablets. The dose of the compound is not particularly limited, since it varies depending on the symptoms; age, sex, body weight and sensitivity of the patient; medication; period and intervals of the administration, properties, composition and kind of the preparation; and kind of the active ingredient. Usually, however, it is given in an amount of about 0.1 to 300 mg, preferably about 1 to 100 mg, per day (1 to 4 times a day).

The compound of the present invention is formulated into injections, suppositories, sublingual tablets, tablets or capsules by an ordinary method employed in the technical field of the formulation.

The injections are prepared by adding, if necessary, a pH adjustor, buffering agent, suspending agent, solubilizer, stabilizer, isotonizing agent and preservative to the active ingredient and they are formulated into the intravenous, subcutaneous or intramuscular injection. If necessary, they can be freeze-dried by an ordinary method.

Examples of the suspending agents include methylcellulose, Polysorbate 80, hydroxyethylcellulose, acacia, tragacanth powder, sodium carboxymethylcellulose and polyoxyethylene sorbitan monolaurate.

Examples of the solubilizers include polyoxyethylene-hardened castor oil, Polysorbate 80, nicotinamide, polyoxyethylene sorbitan monolaurate, macrogol and ethyl esters of castor oil fatty acids.

Examples of the stabilizers include sodium sulfite, sodium metasulfite and ethers. Examples of the preservatives include methyl p-hydroxybenzoate, ethyl p-hydroxybenzoate, sorbic acid, phenol, cresol and chlorocresol.

### [Examples]

The following Example will further illustrate the present invention.

### Example 1

### 3-{2-[1-(1,3-Dioxolan-2-ylmethyl)-4-piperidyl]ethyl}-2H-3,4-dihydro-1,3-6-methoxybenzoxazine-2,4-dione hydrochloride:

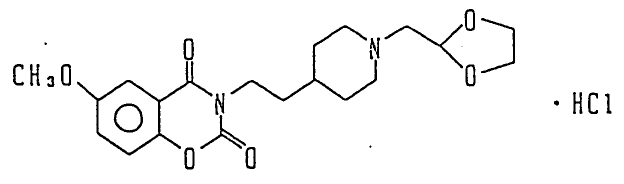

0.99 g of 4-[2-(2-benzyloxy-5-methoxybenzoylamino)ethyl]-1-(1,3-dioxolan-2-ylmethyl)piperidine was dissolved in 30 ml of methanol and 0.11 g of 10% Pd-C was added thereto. The mixture was stirred at room temperature in a hydrogen atmosphere for 2 h. The used Pd-C was removed by filtration and the solvent was distilled off to give 0.82 g of a light yellow oily substance. 30 ml of tetrahydrofuran was added thereto to give a solution and 0.71 g of N,N'-carbonyldiimidazole was added thereto. The mixture was heated under reflux for 20 h. The solvent was distilled off and an oily substance thus obtained was purified by silica gel column chromatography. It was converted into hydrochloride thereof in an ordinary manner and recrystallized from methanol/ether to give 0.85 g of the intended compound as colorless needles.
o Melting point: 155.3 to 156.8°C
o Molecular formula: C₂₀H₂₆N₂O₆·HCl
o NMR(CDCl₃)δ:
   1.35 ∼ 1.43 (3H, m), 1.64 (2H, bq), 1.76 (2H, bd), 2.08 (2H, t, J = 11.0Hz), 2.56 (2H, d, J = 4.4Hz), 3.50 (2H, bd), 3.87 (3H, s), 3.82 ∼ 3.90 (2H, m), 3.92 ∼ 3.99 (2H, m), 4.03 ∼ 4.07 (2H, m), 5.00 (1H, t, J = 4.4Hz), 7.10 (1H, d, J = 9.2Hz), 7.16 (1H, q, J = 2.8Hz, 9.2Hz), 7.35 (1H, d, J = 2.8Hz)
o MS: (M+1⁺) = 391

## Claims (Claims for the following Contracting State(s): DE, GB, FR, IT, NL, SE, LI, CH, BE, AT, LU, DK)

1. A cyclic amide derivative having the formula (Ia) or a pharmacologically acceptable salt thereof:

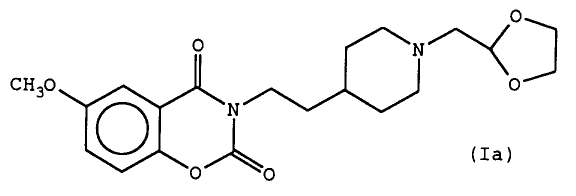

2. A pharmaceutical composition comprising a pharmacologically effective amount of the cyclic amide derivative as defined in claim 1 and a pharmacologically acceptable carrier.

3. The use of the compounds of claim 1 for the preparation of a pharmaceutical useful for preventing and treating diseases due to insufficiency of the central choline functions.

## Claims (Claims for the following Contracting State(s): ES, GR)

1. Method for the production of a compound of formula Ia: 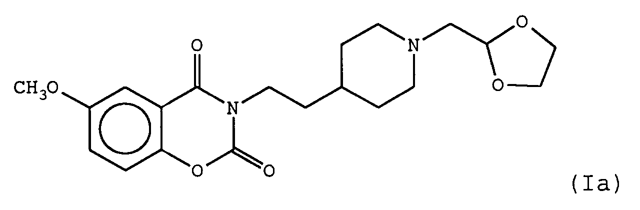 characterized in that it comprises the steps of reacting a compound of the following formula: 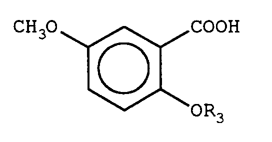 (wherein R₃ represents a protection group, preferably a benzyl, methoxymethyl or methoxyethoxymethyl group) with a further compound of the following formula: 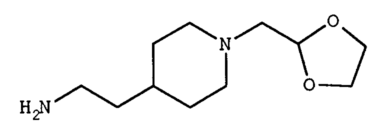 and removing the protecting group, to produce a compound of formula: 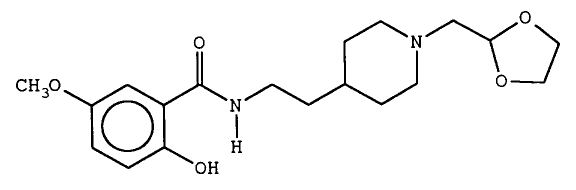 followed by reaction of the latter compound with, for example, 1,1-carbonyldiimidazole to give the target compound, (Ia).

2. The process according to claim 1, wherein R₃ is a benzyl residue.

3. The process according to claims 1 or 2 in which the protective group R₃ is removed by catalytic reduction, preferably in the presence of a palladium/carbon catalyst.

## Patentansprüche (Patentansprüche für folgende(n) Vertragsstaat(en): DE, GB, FR, IT, NL, SE, LI, CH, BE, AT, LU, DK)

1. Zyklisches Amidderivat der Formel (Ia) oder ein pharmakologisch annehmbares Salz davon:

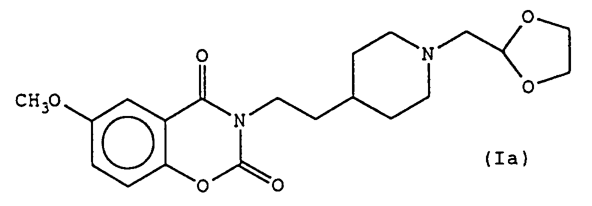

2. Pharmazeutische Zusammensetzung, umfassend eine pharmakologisch wirksame Menge des in Anspruch 1 definierten zyklischen Amidderivats und einen pharmakologisch annehmbaren Träger.

3. Verwendung der Verbindungen des Anspruchs 1 zur Herstellung eines Arzneimittels, das bei der Verhinderung und Behandlung von Krankheiten infolge Insuffizienz der zentralen Cholinfunktionen nützlich ist.

## Patentansprüche (Patentansprüche für folgende(n) Vertragsstaat(en): ES, GR)

1. Verfahren zur Herstellung einer Verbindung der Formel (Ia): 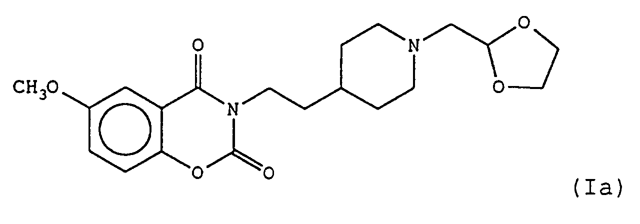 dadurch gekennzeichnet, daß es die Schritte des Umsetzens einer Verbindung mit der folgenden Formel: 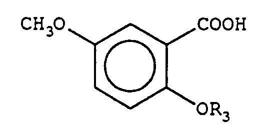 (worin R₃ eine Schutzgruppe, vorzugsweise eine Benzyl-, Methoxymethyl- oder Methoxyethoxymethylgruppe bedeutet) mit einer weiteren Verbindung der folgenden Formel: 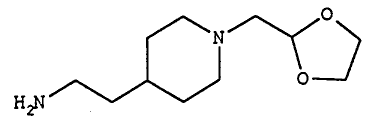 und des Entfernens der Schutzgruppe umfasst, um eine Verbindung der folgenden Formel herzustellen: 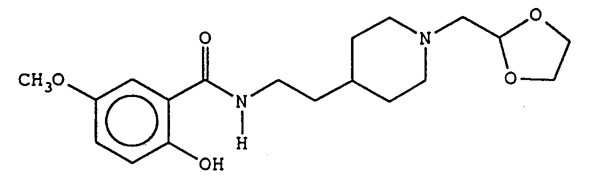 gefolgt von der Umsetzung letzterer Verbindung mit beispielsweise 1,1,-Carbonyldiimidazol, um die Zielverbindung (Ia) zu ergeben.

2. Verfahren gemäß Anspruch 1, worin R₃ ein Benzylrest ist.

3. Verfahren gemäß Anspruch 1 oder 2, in dem die Schutzgruppe R₃ durch katalytische Reduktion, vorzugsweise in Gegenwart eines Palladium/Kohlenstoff-Katalysators entfernt wird.

## Revendications (Revendications pour l'(les) Etat(s) contractant(s) suivant(s): DE, GB, FR, IT, NL, SE, LI, CH, BE, AT, LU, DK)

1. Dérivé d'amide cyclique ayant la formule (Ia) ou un sel pharmacologiquement acceptable de celui-ci:

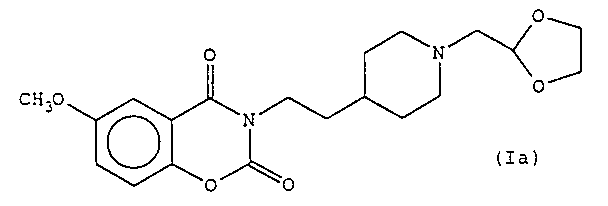

2. Composition pharmaceutique comprenant une quantité pharmacologiquement efficace du dérivé d'amide cyclique tel que défini dans la revendication 1 et un support pharmacologiquement acceptable.

3. Utilisation des composés de la revendication 1 pour la préparation d'un produit pharmaceutique utile pour prévenir et traiter les maladies dues à une insuffisance des fonctions centrales de la choline.

## Revendications (Revendications pour l'(les) Etat(s) contractant(s) suivant(s): ES, GR)

1. Procédé pour la préparation d'un composé de formule Ia: 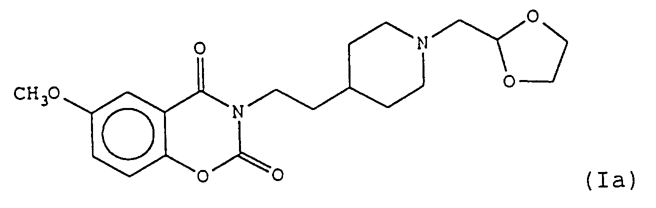 caractérisé en ce que il comprend les étapes de réaction d'un composé ayant la formule suivante: 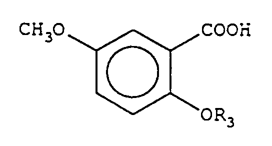 (dans laquelle R₃ représente un groupe de protection, de préférence un groupe benzyle, méthoxyméthyle ou méthoxyéthoxyméthyle) avec un autre composé ayant la formule suivante: 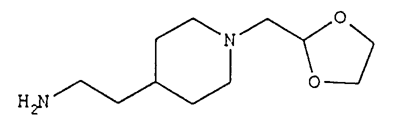 et d'enlèvement du groupe protecteur, pour préparer un composé de formule: 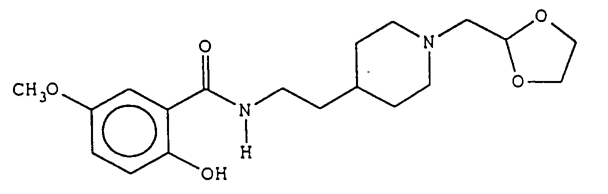 suivi par une réaction de ce dernier composé avec, par exemple, du 1,1-carbonyldiimidazole pour donner le composé cible (Ia).

2. Procédé selon la revendication 1, dans lequel R₃ est un reste benzyle.

3. Procédé selon les revendications 1 ou 2, dans lequel le groupe protecteur R₃ est enlevé par une réduction catalytique, de préférence en présence d'un catalyseur de palladium/carbone.
